# EUROPEAN PATENT SPECIFICATION

(11) **EP 2 495 234 B1**
(45) Date of publication and mention of the grant of the patent: **06.06.2018**
(21) Application number: 10826461.5
(22) Date of filing: 28.09.2010
(51) Int. Cl.: C07C 309/06, C07C 381/12, C08K 5/375, G03F 7/004, C09B 69/00

(54) **AROMATIC SULFONIUM SALT COMPOUND**
AROMATISCHE SULFONIUMSALZVERBINDUNG
COMPOSÉ DE SEL DE SULFONIUM AROMATIQUE

(30) Priority: 26.10.2009 JP 2009245814
(43) Date of publication of application: 05.09.2012
(73) Proprietor: Adeka Corporation, Tokyo 116-8554 (JP)
(72) Inventor: MAKABE, Yoshie, Tokyo 116-8554 (JP); OKUYAMA, Yuta, Tokyo 116-8554 (JP)
(74) Representative: Forstmeyer, Dietmar
(86) International application number: PCT/JP2010/066815
(87) International publication number: WO 2011/052327

(56) References cited:
- EP-A1- 1 036 789
- WO-A1-2004/029037
- WO-A1-2007/003507
- JP-A- 9 278 935
- JP-A- 2000 062 030
- JP-A- 2000 062 030
- JP-A- 2000 186 071
- JP-A- 2000 347 403
- JP-A- 2000 347 403
- JP-A- 2004 137 172
- JP-A- 2008 037 879
- JP-A- 2009 501 148

## Description

### TECHNICAL FIELD

This invention relates to a noble aromatic sulfonium salt compound and more particularly to an aromatic sulfonium salt compound, a photo-acid generator and a cationic polymerization initiator each comprising the compound, and a resist composition and a cationically polymerizable composition containing the photo-acid generator or the cationic polymerization initiator, respectively.

### BACKGROUND ART

A sulfonium salt compound is a substance that generates an acid on exposure to energy radiation, such as light, and is used, for example, as a photo-acid generator in photolithographic resist compositions used in the formation of electronic circuits having semiconductors or as a cationic polymerization initiator in photopolymerizable compositions, such as stereolithographic resin compositions, coatings, and adhesives.

Patent literatures 1 and 2 below each disclose an aromatic sulfonium salt compound, a photopolymerization initiator comprising the compound, an energy radiation-curing composition containing the initiator, and a cured product of the composition. Patent literature 3 below discloses an aromatic sulfonium salt compound, a photo-acid generator comprising the compound, and a photopolymerizable composition containing the photo acid generator. However, these aromatic sulfonium salt compounds disclosed have insufficient solubility and when, in particular, used as a negatively working resist, have difficulty in micropatterning due to insufficient developing properties.

### PRIOR ART DOCUMENT

### PATENT LITERATURE

Patent Literature 1: JP 7-10914A
Patent Literature 2: JP 10-7649A
Patent Literature 3: 2000-186071A (corresponding to U.S. patent 6368769)

### SUMMARY OF THE INVENTION

### PROBLEM TO BE SOLVED BY THE INVENTION

An object of the invention is to provide a photo-acid generator having high developing properties and a resist composition containing the same.

Another object of the invention is to provide a cationic polymerization initiator having high curing properties and a cationically polymerizable composition containing the same.

### MEANS FOR SOLVING THE PROBLEM

As a result of extensive investigations, the inventors have found that an aromatic sulfonium salt compound having a specific structure accomplishes the above objects and thus reached the invention.

The invention provides an aromatic sulfonium salt compound represented by general formula (I): 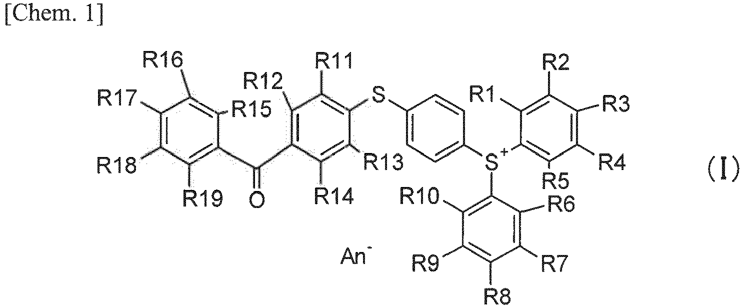
wherein R¹, R², R³, R⁴, R⁵, R⁶, R⁷, R⁸, R⁹, and R¹⁰ each independently represent a hydrogen atom, a halogen atom, a hydroxyl group, an optionally hydroxyl-substituted alkyl group having 1 to 18 carbon atoms, an optionally hydroxyl-substituted ester group having 1 to 12 carbon atoms, an optionally hydroxyl-substituted alkoxy group having 1 to 18 carbon atoms, or an optionally hydroxyl-substituted thioalkoxy group having 1 to 18 carbon atoms;
R¹¹, R¹², R¹³, and R¹⁴ each independently represent a hydrogen atom, a halogen atom, a hydroxyl group, an optionally hydroxyl-substituted alkyl group having 1 to 18 carbon atoms, an optionally hydroxyl-substituted alkoxy group having 1 to 18 carbon atoms, or an optionally hydroxyl-substituted thioalkoxy group having 1 to 18 carbon atoms;
R¹⁵, R¹⁶, R¹⁷, R¹⁸, and R¹⁹ each independently represent a hydrogen atom, a hydroxyl group, a halogen atom, an optionally hydroxyl-substituted alkyl group having 1 to 18 carbon atoms, an optionally hydroxyl-substituted ester group having 1 to 12 carbon atoms, an optionally hydroxyl-substituted alkoxy group having 1 to 18 carbon atoms, or an optionally hydroxyl-substituted thioalkoxy group having 1 to 18 carbon atoms;
An⁻ represents a monovalent anion;
the methylene chain of the optionally hydroxyl-substituted alkyl group having 1 to 18 carbon atoms represented by R¹ through R¹⁹, the optionally hydroxyl-substituted alkoxy group having 1 to 18 carbon atoms represented by R¹ through R¹⁹, and the optionally hydroxyl-substituted thioalkoxy group having 1 to 18 carbon atoms represented by R¹ through R¹⁹ is optionally interrupted by -O-;
wherein at least one of R¹¹ through R¹⁴ is a fluorine atom; and
wherein at least one of R¹ to R¹⁹ is a hydroxyl group or a hydroxyl-substituted thioalkoxy group having 1 to 18 carbon atoms.

The invention also provides a photo-acid generator comprising the aromatic sulfonium salt compound.

The invention also provides a resist composition containing the photo-acid generator.

The invention also provides a cationic polymerization initiator comprising the aromatic sulfonium salt compound.

The invention also provides a cationically polymerizable composition containing the cationic polymerization initiator.

### EFFECT OF THE INVENTION

The aromatic sulfonium salt compound provides a photo-acid generator having high developing properties. A photoresist composition containing the photo-acid generator exhibits high sensitivity and achieves high resolution and is therefore useful as a negative resist sensitive to radiation, such as UV light, electron beam, or X rays, and useful in the fabrication of semiconductor integrated circuits, TFT circuits for LCDs, and masks for circuit formation.

The aromatic sulfonium salt compound of the invention is also useful as a cationic polymerization initiator and provides a cationically polymerizable composition exhibiting excellent curability.

### BEST MODE FOR CARRYING OUT THE INVENTION

The invention will be described in detail based on its preferred embodiments.

The aromatic sulfonium salt compound of the invention will be described first.

The sulfonium salt compound of the invention is a novel compound represented by general formula (I) shown above. One of the characteristics of the sulfonium salt compound of the invention resides in that at least one of R¹ through R¹⁹ is a substituent selected from the group consisting of a hydroxyl group, and a hydroxyl-substituted C1-C18 thioalkoxy group.

In the compound represented by formula (I), examples of the halogen represented by R¹ to R¹⁹ include fluorine, chlorine, bromine, and iodine.

Examples of the optionally hydroxyl-substituted C1-C18 alkyl represented by R¹ to R¹⁹ include methyl, ethyl, propyl, isopropyl, butyl, s-butyl, t-butyl, isobutyl, amyl, isoamyl, t-amyl, hexyl, cyclohexyl, heptyl, octyl, nonyl, ethyloctyl, 2-methoxyethyl, 3-methoxypropyl, 4-methoxybutyl, 2-butoxyethyl, methoxyethoxyethyl, methoxyethoxyethoxyethyl, 3-methoxybutyl, 2-methylthioethyl, fluoromethyl, difluoromethyl, trifluoromethyl, chloromethyl, dichloromethyl, trichloromethyl, bromomethyl, dibromomethyl, tribromomethyl, difluoroethyl, trichloroethyl, dichlorodifluoroethyl, pentafluoroethyl, heptafluoropropyl, nonafluorobutyl, decafluoropentyl, tridecafluorohexyl, pentadecafluoroheptyl, heptadecafluorooctyl, methoxymethyl, 1,2-epoxyethyl, methoxyethyl, methoxyethoxymethyl, methylthiomethyl, ethoxyethyl, butoxymethyl, t-butylthiomethyl, 4-pentenyloxymethyl, trichloroethoxymethyl, bis(2-chloroethoxy)methyl, methoxycyclohexyl, 1-(2-chloroethoxy)ethyl, 1-methyl-1-methoxyethyl, ethyldithioethyl, trimethylsilylethyl, t-butyldimethylsilyloxymethyl, 2-(trimethylsilyl)ethoxymethyl, t-butoxycarbonylmethyl, 4-butoxycarbonylmethyl, ethyloxycarbonylmethyl, ethylcarbonylmethyl, t-butoxycarbonylmethyl, acryloyloxyethyl, methacryloyloxyethyl, 2-methyl-2-adamantyloxycarbonylmethyl, acetylethyl, camphor-10-yl, 2-methoxy-1-propenyl, hydroxylmethyl, 2-hydroxyethyl, 1-hydroxyethyl, 2-hydroxypropyl, 3-hydroxypropyl, 3-hydroxybutyl, 4-hydroxybutyl, and 1,2-dihydroxyethyl.

Examples of the optionally hydroxyl-substituted C1-C12 ester group represented by R¹ to R¹⁰ and R¹⁵ to R¹⁹ include methoxycarbonyl, ethoxycarbonyl, isopropyloxycarbonyl, phenoxycarbonyl, acetoxy, propionyloxy, butyryloxy, chloroacetyloxy, dichloroacetyloxy, trichloroacetyloxy, trifluoroacetyloxy, t-butylcarbonyloxy, methoxyacetyloxy, benzoyloxy, hydroxmethylcarbonyloxy, hydroxymethoxymethylcarbonyloxy, hydroxyethoxyethylcarbonyl, 2-hydroxyethoxycarbonyl, 3 -hydroxypropyloxycarbonyl, 4-hydroxyphenoxycarbonyl, 3-hydroxypropionyloxy, 4-hydroxybutyryloxy, 2,3-dihydroxypropylcarbonyloxy, 4-hydroxybutylcarbonyloxy, 3-hydroxybutylcarbonyloxy, 3-hydroxy-1-methylpropylcarbonyloxy, 5-hydroxypentylcarbonyloxy, 4-hydroxypentylcarbonyloxy, 3-hydroxypentylcarbonyloxy, and 4-hydroxybenzoyloxy.

Examples of the optionally hydroxyl-substituted C1-C18 alkoxy represented by R¹ to R¹⁹ include methoxy, ethoxy, propoxy, isopropoxy, butoxy, s-butoxy, t-butoxy, isobutoxy, pentyloxy, isoamyloxy, t-amyloxy, hexyloxy, cyclohexyloxy, cyclohexylmethyloxy, tetrahydrofuranyloxy, tetrahydropyranyloxy, 2-methoxyethyloxy, 3-methoxypropyloxy, 4-methoxybutyloxy, 2-butoxyethyloxy, methoxyethoxyethyloxy, methoxyethoxyethoxyethyloxy, 3-methoxybutyloxy, 2-methylthioethyloxy, trifluoromethyloxy, hydroxymethyloxy, 1-hydroxyethyloxy, 2-hydroxyethyloxy, 2-hydroxypropyloxy, 3-hydroxypropyloxy, 2,3-dihydroxypropyloxy, 2-hydroxy-1-methylpropyloxy, 2-hydroxy-1-methylethyloxy, 2-hydroxybutyloxy, 4-hydroxybutyloxy, 2,3-dihydroxybutyloxy, 3,4-dihydroxybutyloxy, 2,3,4-trihydroxybutyloxy, 2-(2-hydroxyethyloxy)ethyloxy, 2-hydroxy-3-methoxypropyloxy, 5-hydroxypentyloxy, 2-hydroxy-2-(hydroxymethyl)butyloxy, 3-hydroxy-2-di(hydroxymethyl)propyloxy, 6-hydroxyhexyloxy, 5,6-dihydroxyhexyloxy, 2-hydroxycyclohexyloxy, 4-hydroxycyclohexyloxy, 2,3,4,5,6-pentahydroxycyclohexyloxy, 2-(2-(2-hydroxyethyloxy)ethyloxy)ethyloxy, 4-hydroxymethylcyclohexylmethyloxy, and 2-di(hydroxymethyl)butyloxy.

Examples of the optionally hydroxyl-substituted C1-C18 thioalkoxy represented by R¹ to R¹⁹ include methylthio, ethylthio, propylthio, isopropylthio, butylthio, s-butylthio, t-butylthio, isobutylthio, amylthio, isoamylthio, t-amylthio, hexylthio, cyclohexylthio, adamantylthio, methoxymethylthio, isobutylmethylthio, 2-hydroxyethylthio, 3-hydroxypropylthio, 2,3-dihydroxypropylthio, and 2-hydroxy-1-methylpropylthio.

Examples of the anion represented by An⁻ in formula (I) include halide anions, such as chloride, bromide, iodide, and fluoride; inorganic anions, such as perchlorate, chlorate, thiocyanate, hexafluorophosphate, hexafluoroantimonate, hexafluoroarsenate, and tetrafluoroborate; organic sulfonate anions, such as methanesulfonate, fluorosulfonate, benzenesulfonate, toluenesulfonate, 1-naphthylsulfonate, 2-naphthylsulfonate, trifluoromethanesulfonate, pentafluoroethanesulfonate, heptafluoropropanesulfonate, nonafluorobutanesulfonate, undecafluoropentanesulfonate, tridecafluorohexanesulfonate, pentadecafluoroheptanesulfonate, heptadecafluorooctanesulfonate, perfluoro-4-ethylcyclohexanesulfonate, N-alkyl(or aryl)diphenylamine-4-sulfonate, 2-amino-4-methyl-5-chlorobenzenesulfonate, 2-amino-5-nitrobenenesulfonate, the sulfonate described in JP 2004-53799A, camphorsulfonate, fluorobenzenesulfonate, difluorobenzenesulfonate, trifluorobenzenesulfonate, tetrafluorobenzenesulfonate, and pentafluorobenzenesulfonate; organic phosphate anions, such as octylphosphate, dodecylphosphate, octadecylphosphate, phenylphosphate, nonylphenylphosphate, and 2,2'-methylenebis(4,6-di-t-butylphenyl)phosphonate; organic fluorosulfonimide ions, such as bis(trifluoromethanesulfone)imide ion, bis(pentafluoroethanesulfone)imide ion, bis(heptafluoropropanesulfone)imide ion, bis(nonafluorobutanesulfone)imide ion, bis(undecafluoropentanesulfone)imide ion, bis(pentadecafluoroheptanesulfone)imide ion, bis(tridecafluorohexanesulfone)imide ion bis(heptadecafluorooctanesulfonimide) ion, (trifluoromethanesulfone)(nonafluorobutanesulfone)imide ion, (methanesulfone)(trifluoromethanesulfone)imide ion, and cyclohexafluoropropane-1,3-bis(sulfonyl)imide ion; tetraarylborate anions, such as tetrakis(pentafluorophenyl)borate ion, tetrakis(4-fluorophenyl)borate ion, tetraphenylborate ion, the borate ions described in JP 2008-81470A, JP 2007-112854A, JP 6-184170A (corresponding to U.S. Patent 5468902), JP 2002-526391A (corresponding to U.S. Patent 6747071), and PCT/JP 2008/069562; various aliphatic or aromatic carboxylate anions; and organic sulfonylmethide ions, such as tris(trifluoromethanesulfonyl)methide and tris(methanesulfonyl)methide. Also included are alkylsulfonate ions, fluoro-substituted alkylsulfonate ions, and alkylsulfonimides or fluoro-substituted alkylsulfonimides substituted with acryloyloxy, methacryloyloxy, or aliphatic cycloalkyl, such as norbornyl or adamantyl. If desired, a quencher anion capable of deexciting (quenching) an active molecule in an excited state or a metallocene compound anion of, for example, a ferrocene or ruthenocene compound having an anionic group (e.g., a carboxyl group, a phosphonic acid group, or a sulfonic acid group) on its cyclopentadienyl ring may be used. Of these anions preferred are organic sulfonate anions in view of safety and reactivity (in both deprotection reaction and crosslinking reaction).

Specific examples of the cation of the aromatic sulfonium salt compound of the invention include the following compounds.

Compounds 2, 4, 7, 8, 9, 13, 15, 17, 18 and 22 are reference examples.

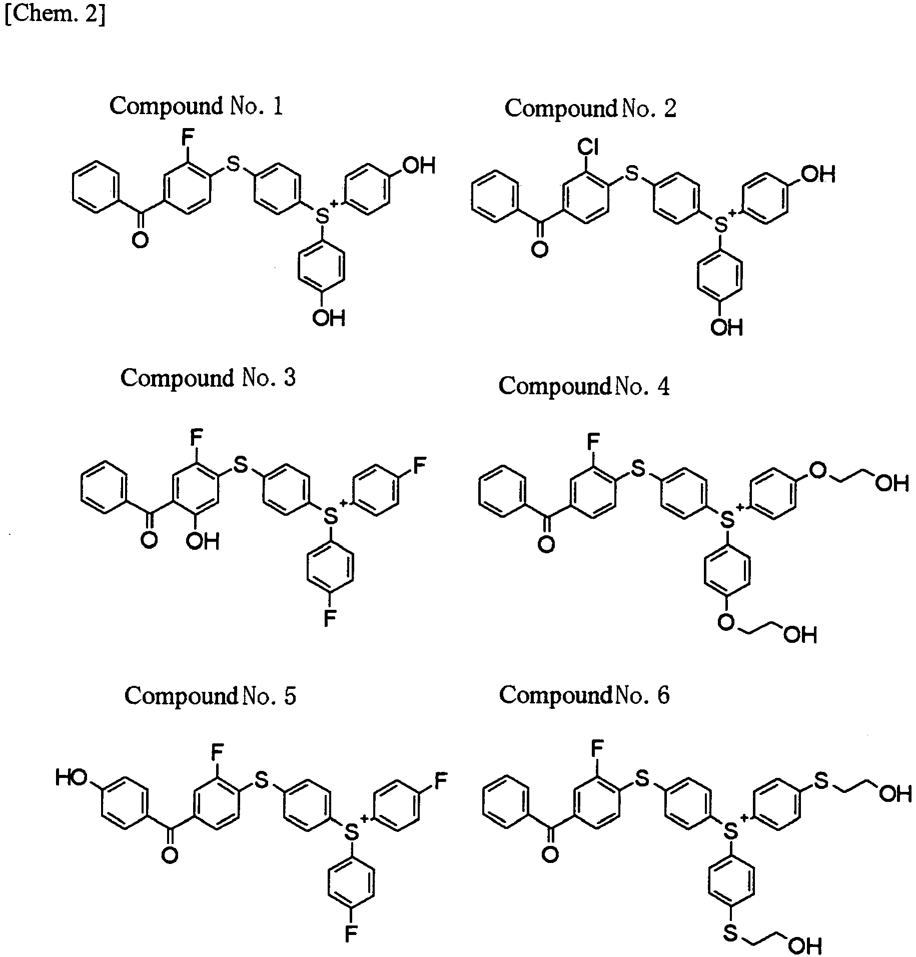

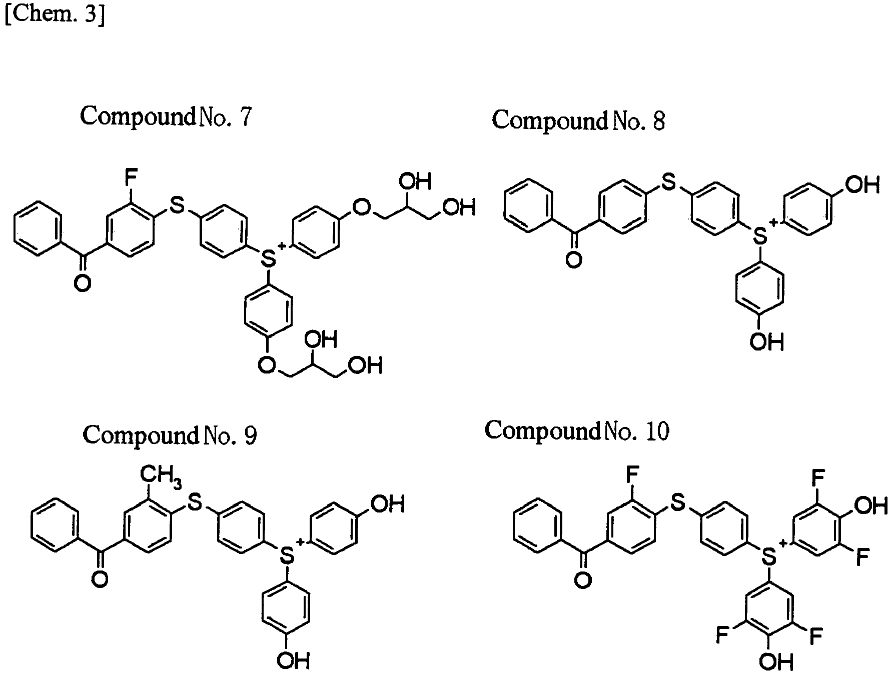

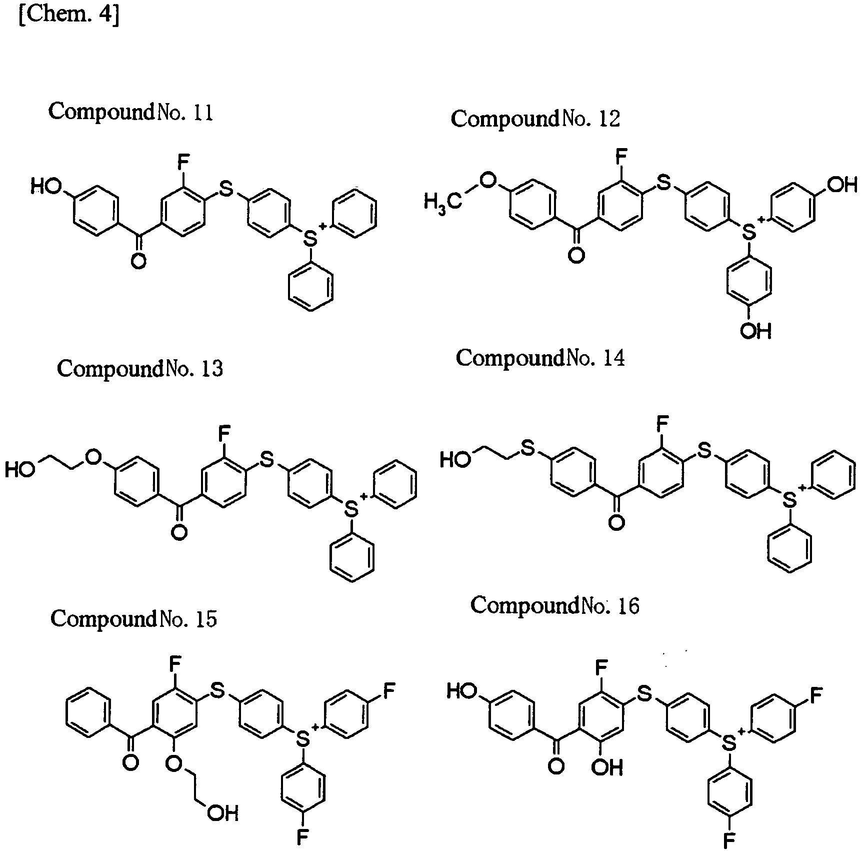

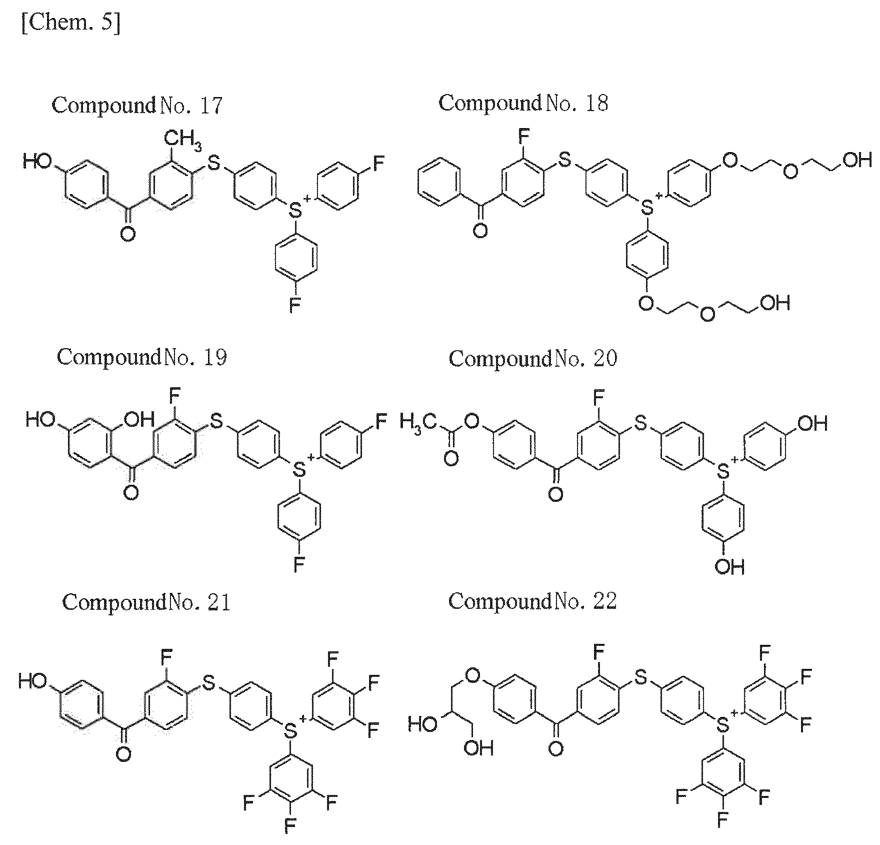

In view of high developing properties and high acid generating ability, more preferred are those compounds in which any one of R¹ to R¹⁰ is hydroxyl, and any one of R¹¹ and R¹⁴ is fluorine; those in which any one of R¹ to R¹⁰ is hydroxyl-substituted C1-C18 alkoxy or hydroxyl-substituted C1-C18 thioalkoxy; and those in which any one of R¹¹ to R¹⁹ is hydroxyl, and any one of R¹¹ to R¹⁴ is fluorine.

The aromatic sulfonium salt compound of the invention may be prepared by any process utilizing known organic synthesis reactions. For example, the aromatic sulfonium salt compound of the invention is obtained by the reaction between a diaryl sulfoxide compound and 4-thiophenylbenzophenone to obtain a sulfonium salt compound, which is, if necessary, subjected to salt exchange with a salt compound having a desired anion component.

The aromatic sulfonium salt compound of the invention has the property of generating a Lewis acid on exposure to active energy radiation, such as extreme ultraviolet light (EUV), X-rays, deep ultraviolet light (DUV) (e.g., F₂, ArF, or KrF laser light, i-line, h-line, or g-line), electron beam, radiation, and high frequency waves, and is capable of acting on an acid-reactive organic substance to induce decomposition or polymerization. Therefore, the sulfonium salt compound of the invention is useful as a photo-acid generator of a positive or negative photoresist or a cationic polymerization initiator.

The photo acid generator of the invention comprises the aromatic sulfonium salt compound of the invention. The photo acid generator of the invention is used in the polymerization of a cationically polymerizable compound, which an acid reactive organic substance, cleavage of the chemical bond (e.g., ester or ether linkage) of acrylic resins, and the like. The amount of the photo acid generator to be used for an acid reactive organic substance is preferably, but not limited to, 0.05 to 100 parts, more preferably 0.05 to 20 parts, by mass per 100 parts by mass of the acid reactive organic substance. The amount may be out of the range recited depending on the properties of the acid reactive organic substance and other factors, such as the irradiation intensity, reaction time, desired physical properties, and cost.

Useful acid reactive organic substances include hereinafter-described resins that change their solubility in a developer by the action of an acid (hereinafter referred to as resist base resins) and stereolithographic resins.

The resist composition according to the invention contains a resist base resin as an acid reactive organic substance and the aromatic sulfonium salt compound of the invention as an essential photo acid generator.

The resist base resin for use in the resist composition of the invention is not particularly limited but is preferably a resin having a small extinction coefficient for the wavelength of active energy radiation used and exhibiting high etching resistance.

Examples of such resist base resins include one or more polymers selected from polyhydroxystyrene and its derivatives; polyacrylic acid and its derivatives; polymethacrylic acid and its derivatives; copolymers obtained from at least two of hydroxystyrene, acrylic acid, methacrylic acid, and their derivatives; copolymers obtained from at least two of hydroxystyrene, styrene, and their derivatives; copolymers obtained from at least three of a cycloolefin and its derivatives, maleic anhydride, and acrylic acid and its derivatives; copolymers obtained from at least three of a cycloolefin and its derivatives, maleimide, and acrylic acid and its derivatives; polynorbornene; and ring-opening metathesis polymers; as well as the polymers enumerated above partially substituted by an acid-labile group showing alkali-solubility controlling ability. Examples of the acid-labile group to be introduced into the polymer include tertiary alkyl, trialkylsilyl, oxoalkyl, aryl-substituted alkyl, alicyclic heterocyclic (e.g., tetrahydropyran-2-yl), tertiary alkylcarbonyl, tertiary alkylcarbonylalkyl, and alkyloxycarbonyl groups.

Detailed description and specific examples of the resist base resin are described, e.g., in claims 8 to 11 of JP 2003-192665A, claim 3 of JP 2004-323704A, and JP 10-10733A.

The polystyrene equivalent weight average molecular weight (Mw) of the resist base resin measured by gel permeation chromatography is usually 1,500 to 300,000, preferably 2,000 to 200,000, even more preferably 3,000 to 100,000. Using a base resin having an Mw of less than 1,500 tends to provide a resist with reduced heat resistance. Using a base resin having an Mw of more than 300,000 tends to provide a resist with reduced developability and coating properties.

The photo acid generator in the resist composition of the invention may contain other photo acid generator in addition to the aromatic sulfonium salt compound of the invention. To secure sensitivity and developability as a resist, the amount of the photo acid generator in the resist composition is usually 0.01 to 20 parts, preferably 0.5 to 10 parts, by mass per 100 parts by mass of the resist base resin. When the amount of the photo acid generator is less than 0.01 parts by mass, the sensitivity and developability of the resulting resist can be reduced. When it is more than 20 parts, the resist can have reduced transparency to radiation, resulting in difficulty in providing a resist pattern having a rectangular cross-section.

Examples of the other photo acid generator that may be used in combination with the aromatic sulfonium salt compound of the invention include iodonium salt compounds and sulfonylimide compounds. The amount of the photo acid generator other than the aromatic sulfonium salt compound, when used in combination, is preferably not more than 50 parts by mass per 100 parts by mass of the aromatic sulfonium salt compound of the invention.

The photo acid generator comprising the aromatic sulfonium salt compound may be compounded into the resist composition of the invention along with various additives as well as the other photo acid generator. Such additives include inorganic fillers, organic fillers, colorants including pigments and dyes, defoaming agents, thickening agents, flame retardants, antioxidants, stabilizers, and leveling agents. The total content of these additives in the resist composition is preferably 50% by mass or less.

On use, the resist composition of the invention is usually adjusted in concentration by diluting with a solvent to a total solids concentration usually of from 5 to 50%, preferably of from 10 to 25%, by weight, followed by filtration through a filter having an opening size of about 0.2 µm. The resist composition of the invention is prepared by mixing the photo acid generator comprising the aromatic sulfonium salt compound, the other photo acid generator, the resist base resins, and the other optional components by dissolving, kneading, or otherwise.

The resist composition of the invention is particularly useful as a chemically amplified resist. Chemically amplified resists are divided into negative resists in which a chemical chain reaction takes place by the action of the acid generated from the photo acid generator on exposure to light to cause the base resin to crosslink or change in polarity to be insolubilized in a developer and positive resists in which the side chain of the base resin is deprotected by the action of the acid to cause the base resin to change in polarity to be solubilized in a developer.

The light that can be used in exposure of the resist composition is selected as appropriate to the photo acid generator used from among visible light, UV light, DUV, X-rays, charged particle radiation, and so on. The invention is advantageously applied to resist compositions that can be patterned by a variety of radiation, such as DUV from a KrF excimer layer (248 nm) or an ArF excimer laser (193 nm), X-rays from synchrotron radiation, and charged particle beams, such as electron beams and EUV.

The cationic polymerization initiator of the invention comprises the aromatic sulfonium salt compound of the invention. The cationically polymerizable composition of the invention contains the cationic polymerization initiator of the invention and a cationically polymerizable compound and is useful in a broad range of application, including photoresists in making lithographic plates, letterpress plates, printed circuit boards, ICs, or LSIs; image formation, such as relief image formation and image replication; and photocuring inks, coatings, or adhesives.

The cationically polymerizable compound for use in the cationically polymerizable composition of the invention is a compound that undergoes polymerization or crosslinking reaction by the action of a cationic polymerization initiator activated on exposure to light. One or more than one cationically polymerizable compounds may be used.

The cationically polymerizable compounds typically include epoxy compounds, oxetane compounds, cyclic lactone compounds, cyclic acetal compounds, cyclic thioether compounds, spiro orthoester compounds, and vinyl compounds. One or more than one cationically polymerizable compounds may be used. *Inter alia,* epoxy compounds and oxetane compounds are suitable in terms of availability and handling convenience.

Suitable examples of the epoxy compounds are alicyclic epoxy compounds, aromatic epoxy compounds, and aliphatic epoxy compounds.

Examples of the alicyclic epoxy compounds include polyglycidyl ethers of polyhydric alcohols having at least one alicyclic ring and cyclohexene oxide- or cyclopentane oxide-containing compounds obtained by epoxidizing cyclohexene ring- or cyclopentane ring-containing compounds with an oxidizing agent. Specific examples thereof are hydrogenated bisphenol A diglycidyl ether, 3,4-epoxycyclohexylmethyl 3,4-epoxycyclohexanecarboxylate, 3,4-epoxy-1-methylcyclohexyl 3,4-epoxy-1-methylcyclohexanecarboxylate, 6-methyl-3,4-epoxycyclohexylmethyl 6-methyl-3,4-epoxycyclohexanecarboxylate, 3,4-epoxy-3-methylcyclohexylmethyl 3,4-epoxy-3-methylcyclohexanecarboxylate, 3,4-epoxy-5-methylcyclohexylmethyl 3,4-epoxy-5-methylcyclohexanecarboxylate, 2-(3,4-epoxycyclohexyl-5,5-spiro-3,4-epoxy)cyclohexane-metadioxane, bis(3,4-epoxycyclohexylmethyl) adipate, 3,4-epoxy-6-methylcyclohexyl carboxylate, methylenebis(3,4-epoxycyclohexane), dicyclopentadiene diepoxide, ethylenebis(3,4-epoxycyclohexane carboxylate), dioctyl epoxyhexahydrophthalate, and di(2-ethylhexyl) epoxyhexahydrophthalate.

Commercially available products that are suitably used as the alicyclic epoxy compound include UVR-6100, UVR-6105, UVR-6110, UVR-6128, and UVR-6200 from Union Carbide; Celloxide 2021, Celloxide 2021P, Celloxide 2081, Celloxide 2083, Celloxide 2085, Celloxide 2000, Celloxide 3000, Cyclomer A200, Cyclomer M100, Cyclomer M101, Epolead GT-301, Epolead GT-302, Epolead 401, Epolead 403, ETHB, and Epolead HD300 all from Daicel Chemical Industries, Ltd.; and KRM-2110 and KRM-2199 from ADEKA Corp.

Preferred of the alicyclic epoxy compounds described above are epoxy resins having a cyclohexene oxide structure in terms of curing properties (cure rate).

Examples of the aromatic epoxy compounds include polyglycidyl ethers of polyhydric phenols having at least one aromatic ring or alkylene oxide adducts thereof, such as glycidyl ethers of bisphenol A, bisphenol F, or an alkylene oxide adduct thereof, and epoxy novolak resins.

Examples of the aliphatic epoxy compounds include polyglycidyl ethers of aliphatic polyhydric alcohols or alkylene oxide adducts thereof, polyglycidyl esters of aliphatic long-chain polybasic acids, homopolymers obtained by vinyl polymerization of glycidyl acrylate or glycidyl methacrylate, and copolymers obtained by vinyl polymerization of glycidyl acrylate or glycidyl methacrylate and other vinyl monomer(s). Typical examples are polyhydric alcohol glycidyl ethers, such as 1,4-butanediol diglycidyl ether, 1,6-hexanediol diglycidyl ether, glycerol triglycidyl ether, trimethylolpropane triglycidyl ether, sorbitol tetraglycidyl ether, dipentaerythritol hexaglycidyl ether, polyethylene glycol diglycidyl ether, and polypropylene glycol diglycidyl ether; polyglycidyl ethers of polyether polyols obtained by adding one or more kinds of alkylene oxides to aliphatic polyhydric alcohols, such as propylene glycol, trimethylolpropane, and glycerol; and diglycidyl esters of aliphatic long-chain dibasic acids. Further included are monoglycidyl ethers of aliphatic higher alcohols, monoglycidyl ethers of phenol, cresol, butylphenol, or polyether alcohols obtained by adding an alkylene oxide thereto, glycidyl esters of higher fatty acids, epoxidized soybean oil, octyl epoxystearate, butyl epoxystearate, and epoxidized polybutadiene.

Commercially available products suitably used as the aromatic or aliphatic epoxy compound include Epikote 801 and Epikote 828 from Yuka Shell Epoxy Co., Ltd.; PY-306, 0163, and DY-022 from Ciba Specialty Chemicals; KRM-2720, EP-4100, EP-4000, EP-4080, EP-4900, ED-505, and ED-506 from ADEKA; Epolite M-1230, Epolite EHDG-L, Epolite 40E, Epolite 100E, Epolite 200E, Epolite 400E, Epolite 70P, Epolite 200P, Epolite 400P, Epolite 1500NP, Epolite 1600, Epolite 80MF, Epolite 100MF, Epolite 4000, Epolite 3002, and Epolite FR-1500 from Kyoeisha Chemical; and Santoto ST0000, YD-716, YH-300, PG-202, PG-207, YD-172, and YDPN638 from Tohto Kasei Co., Ltd.

Examples of the oxetane compounds include 3-ethyl-3-hydroxymethyloxetane, 3-(meta)allyloxymethyl-3-ethyloxetane, (3-ethyl-3-oxetanylmethoxy)methylbenzene, 4-fluoro-[1-(3-ethyl-3-oxetanylmethoxy)methyl]benzene, 4-methoxy-[1-(3-ethyl-3-oxetanylmethoxy)methyl]benzene, [1-(3-ethyl-3 -oxetanylmethoxy)ethyl]phenyl ether, isobutoxymethyl (3-ethyl-3-oxetanylmethyl) ether, isobornyloxyethyl (3-ethyl-3-oxetanylmethyl) ether, isobornyl (3-ethyl-3-oxetanylmethyl) ether, 2-ethylhexyl (3-ethyl-3-oxetanylmethyl) ether, ethyldiethylene glycol (3-ethyl-3-oxetanylmethyl) ether, dicyclopentadiene (3-ethyl-3-oxetanylmethyl) ether, dicyclopentenyloxyethyl (3-ethyl-3-oxetanylmethyl) ether, dicyclopentenyl(3-ethyl-3-oxetanylmethyl) ether, tetrahydrofurfuryl (3-ethyl-3-oxetanylmethyl) ether, tetrabromophenyl (3-ethyl-3-oxetanylmethyl) ether, 2-tetrabromophenoxyethyl (3-ethyl-3-oxetanylmethyl) ether, tribromophenyl (3-ethyl-3-oxetanylmethyl) ether, 2-tribromophenoxyethyl (3-ethyl-3-oxetanylmethyl) ether, 2-hydroxyethyl (3-ethyl-3-oxetanylmethyl) ether, 2-hydroxypropyl (3-ethyl-3-oxetanylmethyl) ether, butoxyethyl (3-ethyl-3-oxetanylmethyl) ether, pentachlorophenyl (3-ethyl-3-oxetanylmethyl) ether, pentabromophenyl (3-ethyl-3-oxetanylmethyl) ether, bornyl (3-ethyl-3-oxetanylmethyl) ether, 3,7-bis(3-oxetanyl)-5-oxa-nonane, 3,3'-(1,3,-(2-methylenyl)propanediyl-bis(oxymethylene)) bis(3-ethyloxetane), 1,4-bis[(3-ethyl-3-oxetanylmethoxy)methyl]benzene, 1,2-bis[(3-ethyl-3-oxetanylmethoxy)methyl]ethane, 1,3-bis[(3-ethyl-3-oxetanylmethoxy)methyl]propane, ethylene glycol bis(3-ethyl-3-oxetanylmethyl) ether, dicyclopentenylbis(3-ethyl-3-oxetanylmethyl) ether, triethylene glycol bis(3-ethyl-3-oxetanylmethyl) ether, tetraethylene glycol bis(3-ethyl-3-oxetanylmethyl) ether, tricyclodecanediyldimethylene (3-ethyl-3-oxetanylmethyl) ether, trimethylolpropane tris(3-ethyl-3-oxetanylmethyl) ether, 1,4-bis(3-ethyl-3-oxetanylmethoxy)butane, 1,6-bis(3-ethyl-3-oxetanylmethoxy)hexane, pentaerythritol tris(3-ethyl-3-oxetanylmethyl) ether, pentaerythritol tetrakis(3-ethyl-3-oxetanylmethyl) ether, polyethylene glycol bis(3-ethyl-3-oxetanylmethyl) ether, dipentaerythritol hexakis(3-ethyl-3-oxetanylmethyl) ether, dipentaerythritol pentakis(3-ethyl-3-oxetanylmethyl) ether, dipentaerythritol tetrakis(3-ethyl-3-oxetanylmethyl) ether, caprolactone-modified dipentaerythritol hexakis(3-ethyl-3-oxetanylmethyl) ether, caprolactone-modified dipentaerythritol pentakis(3-ethyl-3-oxetanylmethyl) ether, ditrimethylolpropane tetrakis(3-ethyl-3-oxetanylmethyl) ether, ethylene oxide-modified bisphenol A bis(3-ethyl-3-oxetanylmethyl) ether, propylene oxide-modified bisphenol A bis(3-ethyl-3-oxetanylmethyl) ether, ethylene oxide-modified hydrogenated bisphenol A bis(3-ethyl-3-oxetanylmethyl) ether, propylene oxide-modified hydrogenated bisphenol A bis(3-ethyl-3-oxetanylmethyl) ether, ethylene oxide-modified bisphenol F (3-ethyl-3-oxetanylmethyl) ether.

Use of the oxetane compound is effective and therefore preferred when, in particular, flexibility is demanded.

Examples of the other cationically polymerizable compounds include cyclic lactone compounds, such as β-propiolactone and ε-caprolactone; cyclic acetal compounds, such as trioxane, 1,3-dioxolane, and 1,3,6-trioxanecyclooctane; cyclic thioether compounds, such as tetrahydrothiophene derivatives; spiro orthoester compounds obtained by the reaction between the above described epoxy compound and a lactone; vinyl compounds, including vinyl ether compounds, such as ethylene glycol divinyl ether, alkyl vinyl ethers, 2-chloroethyl vinyl ether, 2-hydroxyethyl vinyl ether, triethylene glycol divinyl ether, 1,4-cyclohexanedimethanol divinyl ether, hydroxybutyl vinyl ether, and propylene glycol propenyl ether, and ethylenically unsaturated compounds, such as styrene, vinylcyclohexene, isobutylene, and polybutadiene; oxolane compounds, such as tetrahydrofuran and 2,3-dimethyltetrahydrofuran; thiirane compounds, such as ethylene sulfide and thioepichlorohydrin; thietane compounds, such as 1,3-propyne sulfide and 3,3-dimethylthietane; silicones; and other well-known compounds.

The amount of the cationic polymerization initiator comprising the aromatic sulfonium salt compound of the invention is preferably 0.01 to 10 parts by mass, more preferably 0.1 to 5 parts by mass, per 100 parts by mass of the cationically polymerizable compound. When the amount is less than 0.01 part by mass, undercure can result. Using more than 10 parts by mass of the initiator brings about no further increase of effects and can adversely affect the physical properties of the resulting cured product.

In addition to the cationic polymerization initiator comprising the aromatic sulfonium salt compound of the invention and the cationically polymerizable compound, the cationically polymerizable composition may further contain various additives. Useful additives include organic solvents; benzotriazole, triazine, or benzoate UV absorbers; phenol, phosphorus, or sulfur antioxidants; antistatic agents, including cationic, anionic, nonionic, or amphoteric surface active agents; flame retardants, including halogen compounds, phosphoric esters, phosphoric amides, melamine compounds, fluorine resins, metal oxides, melamine (poly)phosphates, and piperazine (poly)phosphate; lubricants including hydrocarbons, fatty acids, aliphatic alcohols, aliphatic esters, aliphatic amides, and metal soaps; colorants including dyes, pigments, and carbon black; silicic acid-based inorganic additives, such as fumed silica, fine silica powder, siliceous stone, diatomaceous earth, clay, kaolin, silica gel, calcium silicate, sericite, kaolinite, flint clay, feldspar powder, vermiculite, attapulgite, talc, mica, minnesotite, pyrophyllite, and silica; and fillers, such as glass fiber and calcium carbonate; crystallizing agents including nucleating agents and crystallization accelerators; silane coupling agents; rubber elasticity imparting agents, such as flexible polymers; and sensitizers. The total content of these additives in the cationically polymerizable composition of the invention is preferably up to 50% by mass.

To facilitate dissolving the cationic polymerization initiator in the cationically polymerizable compound, the cationic polymerization initiator may previously be dissolved in an appropriate solvent (e.g., propylene carbonate, carbitol, carbitol acetate, or butyrolactone). The cationically polymerizable composition of the invention is prepared by mixing the cationic polymerization initiator comprising the aromatic sulfonium salt compound of the invention, the cationically polymerizable compound, and other optional components by dissolving, kneading, or a like means.

The cationically polymerizable composition of the invention cures on exposure to energy radiation, such as UV light, to become dry to the touch or solvent-insoluble usually in 0.1 second to several minutes. While any energy radiation capable of inducing decomposition of the cationic polymerization initiator may be used as appropriate, it is preferred to use electromagnetic energy radiation having a wavelength of 2000 to 7000 Å emitted from an ultrahigh, high, medium, or low pressure mercury lamp, a xenon lamp, a carbon arc lamp, a metal halide lamp, a fluorescent lamp, a tungsten lamp, an excimer lamp, a germicidal lamp, an excimer laser, a nitrogen laser, an argon ion laser, a helium cadmium laser, a helium neon laser, a krypton ion laser, various semiconductor lasers, a YAG laser, a light emitting diode, an CRT, or a like light source; or high energy radiation, such as an electron beam, an X-ray, or a radiation.

An exposure time of about 0.1 to 10 seconds will generally be sufficient, while varying according to the energy radiation intensity, the coating thickness, and the cationically polymerizable organic compound. A longer exposure time would be recommended in curing a relatively thick coating. In 0.1 seconds to several minutes after the exposure, most of the compositions become dry to the touch as a result of cationic polymerization. In some cases, it is advantageous to use thermal energy by heating or from a thermal head in combination to accelerate the cationic polymerization.

### EXAMPLES

Examples 1-1 to 1-6 show preparation of a trifluoromethanesulfonate and a hexafluoroantimonate of compound No. 1 and a trifluoromethanesulfonate of each of compound Nos. 3 to 6. Examples 2-1 and 2-2 and Comparative Example 2-1 show evaluation on solubility in an alkaline developer of an alkali developable negative resist containing a trifluoromethanesulfonate of each of compound Nos. 1 and 5 or comparative compound No. 1. Example 3 demonstrates preparation and curability evaluation of a negative resist composition (cationically polymerizable composition) containing a hexafluoroantimonate each of compound Nos. 1, 3, 4, and 5 as a photo acid generator (cationic polymerization initiator).

### Example 1-1: Synthesis of trifluoromethanesulfonate of compound No. 1

A 200 ml four-necked flask was charged with 72.11 g (0.735 mol) of concentrated sulfuric acid. After purging with nitrogen, the contents were cooled to an inner temperature of 10°C. In the flask was put 11.24 g (0.048 mol) of bis(hydroxyphenyl) sulfoxide, and a solution of 12.33 g (0.04 mol) of 3-fluoro-4-phenylthio-benzophenone in 12.33 g of chlorobenzene was added thereto dropwise, followed by stirring at an inner temperature of 30°C for 3 hours. The reaction mixture was poured into a previously prepared mixture of 120 g of ice water, 120 g of methanol, and 120 g of toluene in a 1 L beaker, stirred for 1 hour, and left to stand. The upper layer was discarded. To the lower layer were added 200 ml of methylene chloride and 7.49 g (0.048 mol) of lithium trifluoromethanesulfonate, followed by stirring for 1.5 hours. The methylene chloride layer was washed with three 120 g portions of water, and the solvent was removed by evaporation. The resulting crude product was purified by silica gel column chromatography using methylene chloride/acetone/methanol (8/1/1) to give 7.61 g of a trifluoromethanesulfonate of compound No. 1 (yield: 25.0%; HPLC purity: 94.4%).

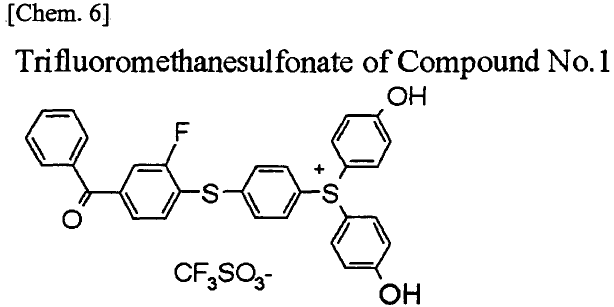

### Example 1-2: Synthesis of hexafluoroantimonate of compound No. 1

A hexafluoroantimonate of compound No. 1 was obtained in the same manner as in Example 1-1, except for replacing lithium trifluoromethanesulfonate with 13.19 g (0.048 mol) of KSbF₆ (yield: 6.49 g, 21.3%; HPLC purity: 93.2%).

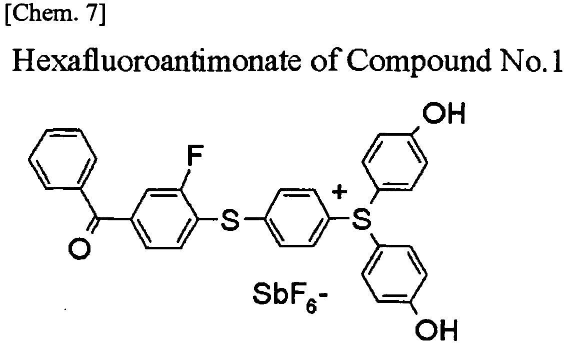

### Example 1-3: Synthesis of trifluoromethanesulfonate of compound No. 3

### Step 1: Synthesis of 3,4-difluoro-6-methoxybenzophenone

A 200 ml four-necked flask was charged with 28.00 ml of tetrachloroethane, 15.97 g (0.111 mol) of 3,4-difluoroanisole, 0.50 g (3.67×10⁻³ mol) of zinc chloride, and 20.00 g (0.142 mol) of benzoyl chloride, followed by stirring. After purging with nitrogen, the mixture was heated at an inner temperature of 150°C for 8 hours. The reaction mixture was poured into a previously prepared mixture of 200 ml of toluene and 100 ml of water in a 1 L beaker. To the toluene layer was added 50 ml of water, and the pH was adjusted to 10 with NaOH. The system was further washed with four 50 ml portions of water. The toluene layer was concentrated, and 20 ml of hexane was added to the concentrate, whereupon crystals precipitated. The crystals were collected by filtration and dried to give 9.00 g of 3,4-difluoro-6-methoxybenzophenone (yield: 32.7%; HPLC purity: 98.1%).

### Step 2: Synthesis of 3-fluoro-6-methoxy-4-phenylthiobenzophenone

In a 50 ml four-necked flask were put 2.73 g (0.011 mol) of the compound obtained in step 1, 6.00 g of dimethylformamide (DMF), and 1.45 g (0.0132 mol) of thiophenol and stirred. After purging with nitrogen, 1.37 g (0.0165 mol) of a 48% NaOH aqueous solution was added thereto dropwise at 20 to 25°C, followed by stirring for 1 hour. To the reaction mixture was added 40 ml of methylene chloride and washed with five 50 ml portions of water. The solvent was removed from the methylene chloride layer by evaporation. The residue was dissolved in 1.0 ml of toluene, and 5.0 ml of hexane was added thereto. The crystals thus precipitated were collected by filtration and dried to furnish 3.22 g of 3-fluoro-6-methoxy-4-phenylthiobenzophenone (yield: 86.6%; HPLC purity: 98.9%).

### Step 3: Synthesis of intermediate No. 3

A 50 ml four-necked flask was charged with 16.87 g of concentrated sulfuric acid. After purging with nitrogen, the contents were cooled to an inner temperature of 10°C. A solution of 2.68 g (0.0112 mol) of bis(fluorophenyl) sulfoxide in 2.68 g of chlorobenzene was added thereto dropwise, and a solution of 3.17 g (0.00936 mol) of the compound obtained in step 2 in 6.20 g of chlorobenzene was then added dropwise, followed by stirring at 35°C for 2.5 hours. The reaction mixture was poured into a previously prepared mixture of 40 ml of toluene, 40 ml of methanol, and 40 ml of water in a 500 ml beaker, followed by stirring for 1 hour. The upper layer was discarded. To the lower layer were added 50 ml of methylene chloride and 1.752 g (0.0112 mol) of lithium trifluoromethanesulfonate, followed by stirring for 1 hour. The methylene chloride layer was washed with four 50 ml portions of water. Removal of the solvent by evaporation yielded 5.52 g of intermediate No. 3 (yield: 83.2%; HPLC purity: 82.1%).

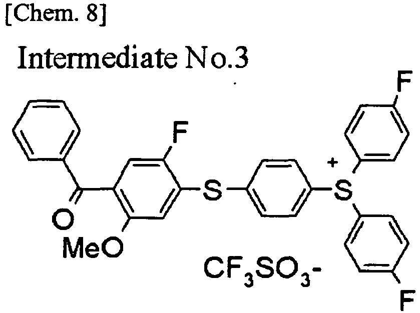

### Step 4: Synthesis of trifluoromethanesulfonate of compound No. 3

A 50 ml four-necked flask was charged with 4.57 g (0.00645 mol) of intermediate No. 3 obtained in step 3, 13.5 g of tetrachloroethane, and 4.30 g (0.0322 mol) of aluminum chloride, followed by stirring, followed by purging with nitrogen. To the mixture was added 0.944 g (0.00646 mol) of octanethiol, followed by stirring at an inner temperature of 30°C for 6 hours. The reaction mixture was poured into a previously prepared mixture of 200 ml of methylene chloride and 50 ml of water in a 500 ml beaker, followed by stirring for 1 hour. The upper layer was discarded. The methylene chloride layer was adjusted to pH of 7 to 8 by the addition of 50 ml of water and an NaOH aqueous solution, washed with three 50 ml portions of water, and concentrated. The resulting crude product was purified by silica gel column chromatography (methylene chloride/acetone=7/3) to afford 2.32 g of a trifluoromethanesulfonate of compound No. 3 (yield: 51.8%; HPLC purity: 93.2%).

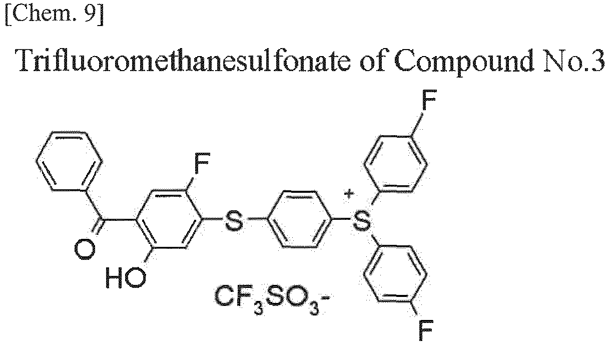

### Example 1-4: Synthesis of trifluoromethanesulfonate of compound No. 4 (reference example)

### Step 1: Synthesis of intermediate No. 4

A 200 ml four-necked flask was charged with 99.06 g (1.01 mol) of concentrated sulfuric acid. After purging with nitrogen, the contents were cooled to an inner temperature of 10°C, and a solution of 16.38 g (0.0688 mol) of bis(fluorophenyl) sulfoxide in 16.38 g of chlorobenzene was added thereto dropwise, followed by dropwise addition of a solution of 16.96 g (0.055 mol) of 3-fluoro-4-phenylthio-benzophenone in 33.00 g of chlorobenzene, followed by stirring at an inner temperature of 30°C for 3 hours. The reaction mixture was poured into a previously prepared mixture of 120 g of ice water, 120 g of methanol, and 120 g of toluene in a 1 L beaker, stirred for 1 hour, and allowed to stand. The upper layer was discarded. To the lower layer were added 200 ml of methylene chloride and 10.73 g (0.0688 mol) of lithium trifluoromethanesulfonate, followed by stirring for 1 hour. The methylene chloride layer was washed with three 120 g portions of water. The solvent was removed by evaporation, and the resulting crude product was purified by silica gel column chromatography (methylene chloride/acetone/methanol = 8/1/1) to give 23.74 g of intermediate No. 4 (yield: 63.6%; HPLC purity: 96.9%).

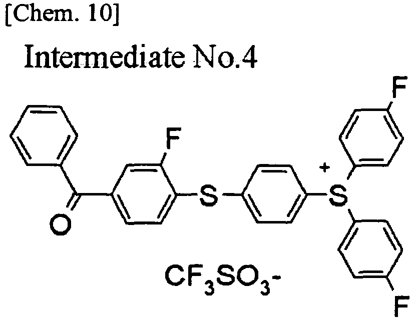

### Step 2: Synthesis of trifluoromethanesulfonate of compound No. 4

A 50 ml four-necked flask was charged with 3.40 g (5.01×10⁻³ mol) of intermediate No. 4 obtained in step 1, 4.00 g of DMF, and 3.20 g (0.0516 mol) of ethylene glycol, followed by stirring, followed by purging with nitrogen. To the mixture was dropwise added 1.04 g (0.0125 mol) of a 48% NaOH aqueous solution, followed by stirring for 3 hours. To the reaction mixture was added 30 ml of methylene chloride, and 60 ml of water and HCl were then added to adjust to a pH of 2. The methylene chloride layer was washed with three 60 ml portions of water, and the solvent was removed by evaporation. The resulting crude product was purified by silica gel column chromatography (methylene chloride/acetone/methanol = 6/3/1) to afford 1.81 g of a trifluoromethanesulfonate of compound No. 4 (yield: 47.4%; HPLC purity: 94.6%).

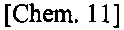

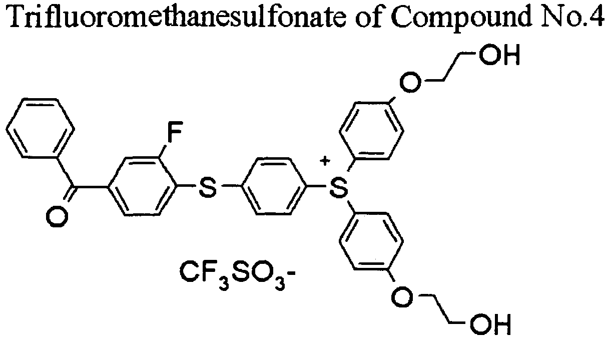

### Example 1-5: Synthesis of trifluoromethanesulfonate of compound No. 5

### Step 1: Synthesis of 3,4-difluoro-4'-methoxybenzophenone

A 100 ml four-necked flask was charged with 16.65 g (0.154 mol) of anisole, 35.0 g of tetrachloroethane, 24.72 g (0.14 mol) of 3,4-difluorobenzoyl chloride, and 0.630 g (0.033 mol) of zinc chloride, followed by stirring. After purging with nitrogen, the system was further stirred at 135°C for 4 hours. The reaction mixture was poured into a previously prepared mixture of 200 ml of toluene and 100 ml of water in a 1 L beaker and stirred. To the toluene layer was added 100 ml of water, and the mixture was adjusted to a pH of 10 with NaOH and washed with four 100 ml portions of water. Concentration of the toluene layer and addition of 100 ml of hexane resulted in precipitation of crystals, which were collected by filtration and dried to give 13.63 g of 3,4-difluoro-4'-methoxybenzophenone (yield: 39.2%; HPLC purity: 97.8%).

### Step 2: Synthesis of 3-fluoro-4'-methoxy-4-phenylthiobenzophenone

In a 200 ml four-necked flask were put 13.55 g (0.0546 mol) of the compound obtained in step 1, 30.00 g of DMF, and 7.219 g (0.0655 mol) of thiophenol and stirred. After purging with nitrogen, 6.82 g (0.0819 mol) of a 48% NaOH aqueous solution was added thereto dropwise at 20 to 25°C, followed by stirring for 1 hour. To the reaction mixture was added 100 ml of methylene chloride, and the mixture was washed with five 100 ml portions of water. The solvent was removed from the methylene chloride layer by evaporation. The residue was dissolved in 6.0 ml of toluene, and 50 ml of hexane was added thereto. The crystals thus precipitated were collected by filtration and dried to give 16.60 g of 3-fluoro-4'-methoxy-4-phenylthiobenzophenone (yield: 89.8%; HPLC purity: 99.6%).

### Step 3: Synthesis of intermediate No. 5

A 200 ml four-necked flask was charged with 87.43 g of concentrated sulfuric acid. After purging with nitrogen, the contents were cooled to an inner temperature of 10°C. A solution of 13.87 g (0.0582 mol) of bis(fluorophenyl) sulfoxide in 13.87 g of chlorobenzene was added thereto dropwise, and a solution of 16.41 g (0.0485 mol) of the compound obtained in step 2 in 32.82 g of chlorobenzene was then added dropwise, followed by stirring at 35°C for 2.5 hours. The reaction mixture was poured into a previously prepared mixture of 200 ml of toluene, 150 ml of methanol, and 200 ml of water in a 1 L beaker, followed by stirring for 1 hour. The upper layer was discarded. To the lower layer were added 150 ml of methylene chloride and 9.08 g (0.0582 mol) of lithium trifluoromethanesulfonate, followed by stirring for 1 hour. The methylene chloride layer was washed with four 200 ml portions of water. Removal of the solvent by evaporation gave 34.25 g of intermediate No. 5 (yield: 99.7%; HPLC purity: 93.8%).

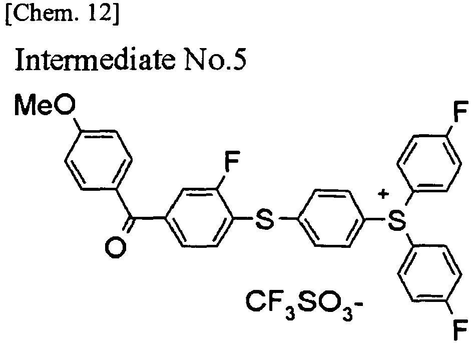

### Step 4: Synthesis of trifluoromethanesulfonate of compound No. 5

A 50 ml four-necked flask was charged with 15.31 g (0.0216 mol) of intermediate No. 5 obtained in step 3, 35.00 g of tetrachloroethane, and 14.12 g (0.106 mol) of aluminum chloride, followed by stirring, followed by purging with nitrogen. To the mixture was added 0.775 g (0.0106 mol) of octanethiol, followed by stirring at an inner temperature of 30°C for 10 hours. The reaction mixture was poured into a previously prepared mixture of 80 ml of methylene chloride and 80 ml of water in a 500 ml beaker, followed by stirring for 1 hour. The upper layer was discarded, and 70 ml of water and an NaOH aqueous solution were added to the methylene chloride layer to adjust to a pH of 7 to 8. The methylene chloride layer was washed with three 80 ml portions of water and concentrated. The resulting crude product was purified by silica gel column chromatography (methylene chloride/acetone = 7/3) to afford 4.79 g of a trifluoromethanesulfonate of compound No. 5 (yield: 31.9%; HPLC purity: 97.3%).

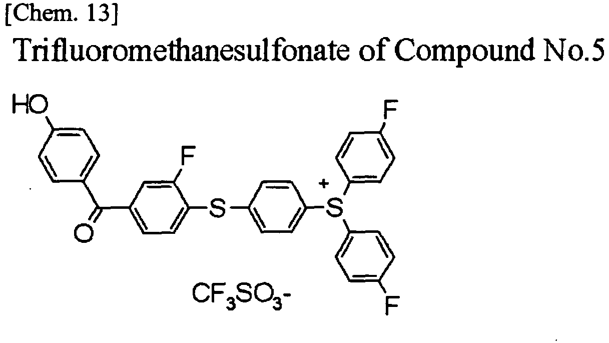

### Example 1-6: Synthesis of trifluoromethanesulfonate of compound No. 6

A 50 ml four-necked flask were charged with 3.40 g (5.01×10⁻³ mol) of intermediate No. 4 and 8.00 g of DMF, followed by stirring to dissolve. To the solution was added 1.73 g (0.0125 mol) of K₂CO₃, followed by stirring. After purging with nitrogen, 0.978 g (0.0125 mol) of 2-mercaptoethanol was added thereto dropwise, followed by stirring at room temperature for 2 hours. The reaction mixture was poured into 30 ml of methylene chloride, and 60 ml of water was added thereto. The methylene chloride layer was washed with four 60 ml portions of water, and the solvent was removed by evaporation. The resulting crude product was purified by silica gel column chromatography (methylene chloride/acetone/methanol = 6/3/1) to afford 2.11 g of a trifluoromethanesulfonate of compound No. 6 (yield: 53.0%; HPLC purity: 92.9%).

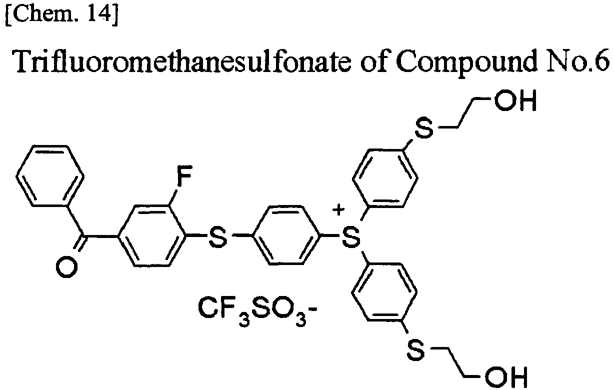

The ¹H and ¹⁹F NMR spectral data of the trifluoromethanesulfonate and the hexafluoroantimonate of compound No. 1 and the trifluoromethanesulfonate of each of compound Nos. 3 to 6 obtained above are shown in Table 1 below. The position of attachment in each compound was determined by cosy, HMQC, and HMBC NMR using the solvent shown in Table 1.

**[Table 1]**

| | |
|---|---|
| Trifluoromethanesulfonate of Compound No.1 DMSO-d6 | 11.3-10.7(s,2H), 7.78-7.81(m,2H), 7.69-7.72(m,3H), 7.56-7.65(m,11H), 7.08(d, 4H) |
| | -77.30(3H), -106.7(1H) |
| Hexafluoroantimonate of Compound No.1 DMSO-d6 | 11.4-10.8(s,2H), 7.77-7.82(m,2H), 7.69-7.73(m,3H), 7.57-7.65(m, 11H), 7.13(d,4H) |
| | -106.54 |
| Trifluoromethanesulfonate of Compound No.3 CDCl₃ | 11.70(s,1H), 7.88(m,4H), 7.74(d,2H), 7.69(d, 2H), 7.63(t,1H), 7.52-7.58(m,4H), 7.35-7.42(m, 5H), 7.04(d,1H) |
| | -78.05(3H), -99.65(2H),- 118.95(1H) |
| Trifluoromethanesulfonate of Compound No.4 DMSO | 7.79(d,2H), 7.68(d,4H), 7.56-7.63(m,6H), 7.51(t,2H), 7.43(d,2H), 7.31(d,4H), 4.94(t,2H), 4.11(t,4H), 3.73(m,4H) |
| | -77.55(3H), -106.28(1H) |
| Trifluoromethanesulfonate of Compound No.5 Acetone-d6 | 8.07(m,4H), 7.88(d,2H), 7.78(d,1H), 7.73(d,2H), 7.59-7.65(m,8H), 7.03(d,2H) |
| | -78.48(3H), -103.47(2H), -106.76(1H) |
| Trifluoromethanesulfonate of Compound No.6 DMSO | 7.81(d,2H), 7.70(d,4H), 7.55-7.61(m,6H), 7.52(t,2H), 7.49(d,4H), 7.41(d,2H), 5.05(t,2H), 4.05(t,4H), 3.72(m,4H) |
| | -77.61 (3H), -106.32(1H) |

The trifluoromethanesulfonate and the hexafluoroantimonate of compound No. 1 and the trifluoromethanesulfonate of each of compound Nos. 3 to 6 were further analyzed by TOF-MS under the following conditions to determine the masses of the compound component and the anion component (positive/negative). The results obtained are shown in Table 2.

### Measuring conditions:

Instrument: Voyager-DE STR
Matrix: Dithranol (Mw: 226.2)
Instrument mode: Reflector

### Positive mode and negative mode

Each of the test compounds and the matrix was formulated into a 1% THF solution, and the test compound solution and the matrix solution were used in a ratio of 2 µl:20 µl.

**[Table 2]**

| | TOF-MS (positive/negative |
|---|---|
| Trifluoromethanesulfonate of Compound No. 1 | 525.2/149.0 |
| Hexafluoroantimonate of Compound No.1 | 525.3/234.9, 236.9 |
| Trifluoromethanesulfonate of Compound No.3 | 545.4/149.0 |
| Trifluoromethanesulfonate of Compound No.4 | 613.4/149.0 |
| Trifluoromethanesulfonate of Compound No.5 | 545.4/149.0 |
| Trifluoromethanesulfonate of Compound No.6 | 645.3/149.0 |

### Examples 2-1 and 2-2 and Comparative Example 2-1 - Evaluation on solubility in polyvinylphenol/tetramethylammonium hydroxide (TMAH) aqueous solution

In a screw-top tube were put 0.36 g of polyvinylphenol (S-2P from Maruzen Petrochemical Co., Ltd.; Mw: 5800) and 10.00 g of a 3% TMAH aqueous solution and heated to dissolve. A 2.00 g portion of the resulting solution was put in another screw-top tube, and a small amount of each of the trifluoromethanesulfonate of each of compound Nos. 1 and 5 and comparative compound No. 1 (intermediate No. 4 obtained in step 1 of Example 1-4) was added thereto and heated. A visual check was made to see whether the compound dissolved. This operation was repeated until saturation. The saturated solution was allowed to stand one day to make sure that no solid precipitated. The mass ratio of each compound to the polyvinylphenol of the saturated solution was taken as a parameter of solubility. The results obtained are shown in Table 3.

**[Table 3]**

| | Compound | Solubility (%) |
|---|---|---|
| Example 2-1 | Trifluoromethanesulfonate of Compound No.1 | 70 |
| Example 2-2 | Trifluoromethanesulfonate of Compound No.5 | 28 |
| Comp. Example 1-1 | Comp. Compound No. 1 | 1> |

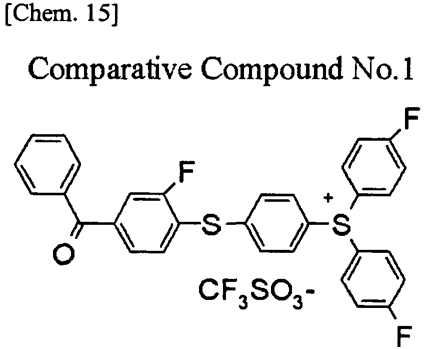

It is apparent from the results in Table 3 that the aromatic sulfonium salt compound of the invention has much higher solubility in a polyvinylphenol/TMAH aqueous solution than comparative compound No. 1 and superior in developing properties.

### Example 3 - Preparation and evaluation of negative resist composition

A hexafluoroantimonate of each of compound Nos. 3, 4, and 5 was synthesized. A resin solution was prepared by dissolving 100 g of EPPN-201 from Nippon Kayaku in 100 g of methyl ethyl ketone (MEK). A 0.05 g portion of each of the hexafluoroantimonate salts was dissolved in 8.00 g of the resin solution to obtain a resist solution. The resist solution was applied to an aluminum plate with a #9 bar coater and dried at 80°C for 10 minutes. The resist was patternwise exposed to light of 365 nm at an irradiance of 65 mW/cm² for 9 seconds, baked at 80°C for 10 minutes, and immersed in MEK for 30 seconds. Visual observation of the coating revealed no peeling.

The results of Example 3 confirm that a resist composition (cationically polymerizable composition) containing the aromatic sulfonium salt compound of the invention as a photo acid generator (cationic polymerization initiator) exhibits high curability and is particularly superior as a negative resist composition.

## Claims

1. An aromatic sulfonium salt compound represented by general formula (I): 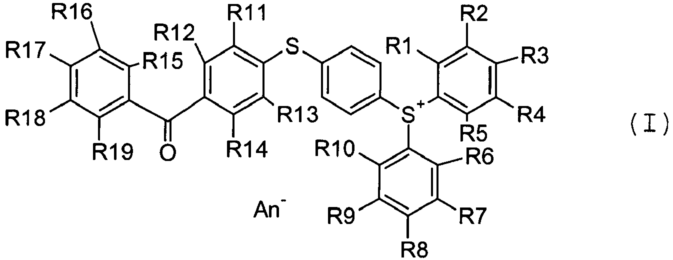
wherein R¹, R², R³, R⁴, R⁵, R⁶, R⁷, R⁸, R⁹, and R¹⁰ each independently represent a hydrogen atom, a halogen atom, a hydroxyl group, an optionally hydroxyl-substituted alkyl group having 1 to 18 carbon atoms, an optionally hydroxyl-substituted ester group having 1 to 12 carbon atoms, an optionally hydroxyl-substituted alkoxy group having 1 to 18 carbon atoms, or an optionally hydroxyl-substituted thioalkoxy group having 1 to 18 carbon atoms;
R¹¹, R¹², R¹³, and R¹⁴ each independently represent a hydrogen atom, a halogen atom, a hydroxyl group, an optionally hydroxyl-substituted alkyl group having 1 to 18 carbon atoms, an optionally hydroxyl-substituted alkoxy group having 1 to 18 carbon atoms, or an optionally hydroxyl-substituted thioalkoxy group having 1 to 18 carbon atoms;
R¹⁵, R¹⁶, R¹⁷, R¹⁸, and R¹⁹ each independently represent a hydrogen atom, a hydroxyl group, a halogen atom, an optionally hydroxyl-substituted alkyl group having 1 to 18 carbon atoms, an optionally hydroxyl-substituted ester group having 1 to 12 carbon atoms, an optionally hydroxyl-substituted alkoxy group having 1 to 18 carbon atoms, or an optionally hydroxyl-substituted thioalkoxy group having 1 to 18 carbon atoms;
An⁻ represents a monovalent anion;
the methylene chain of the optionally hydroxyl-substituted alkyl group having 1 to 18 carbon atoms represented by R¹ through R¹⁹, the optionally hydroxyl-substituted alkoxy group having 1 to 18 carbon atoms represented by R¹ through R¹⁹, and the optionally hydroxyl-substituted thioalkoxy group having 1 to 18 carbon atoms represented by R¹ through R¹⁹ is optionally interrupted by -O-;
wherein at least one of R¹¹ through R¹⁴ is a fluorine atom; and
wherein at least one of R¹ to R¹⁹ is a hydroxyl group or a hydroxyl-substituted thioalkoxy group having 1 to 18 carbon atoms.

2. A photo-acid generator comprising the aromatic sulfonium salt compound according to claim 1.

3. A resist composition containing the photo-acid generator according to claim 2.

4. A cationic polymerization initiator comprising the aromatic sulfonium salt compound according to claim 1.

5. A cationically polymerizable composition containing the cationic polymerization initiator according to claim 4.

## Patentansprüche

1. Aromatische Sulfoniumsalzverbindung der allgemeinen Formel (I) : 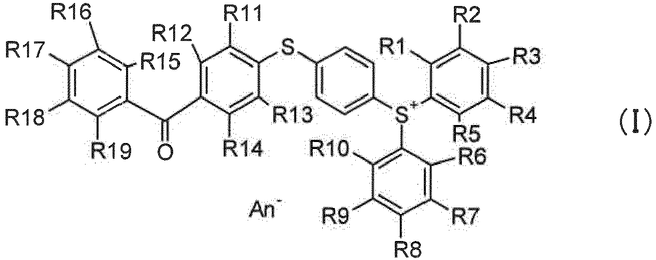
wobei R¹, R², R³, R⁴, R⁵, R⁶, R⁷, R⁸, R⁹ und R¹⁰ jeweils unabhängig voneinander ein Wasserstoffatom, ein Halogenatom, eine Hydroxylgruppe, eine gegebenenfalls durch Hydroxyl substituierte Alkylgruppe mit 1 bis 18 Kohlenstoffatomen, eine gegebenenfalls durch Hydroxyl substituierte Estergruppe mit 1 bis 12 Kohlenstoffatomen, eine gegebenenfalls durch Hydroxyl substituierte Alkoxygruppe mit 1 bis 18 Kohlenstoffatomen oder eine gegebenenfalls durch Hydroxyl substituierte Thioalkoxygruppe mit 1 bis 18 Kohlenstoffatomen darstellen;
R¹¹, R¹², R¹³ und R¹⁴ jeweils unabhängig voneinander ein Wasserstoffatom, ein Halogenatom, eine Hydroxylgruppe, eine gegebenenfalls durch Hydroxyl substituierte Alkylgruppe mit 1 bis 18 Kohlenstoffatomen, eine gegebenenfalls durch Hydroxyl substituierte Alkoxygruppe mit 1 bis 18 Kohlenstoffatomen oder eine gegebenenfalls durch Hydroxyl substituierte Thioalkoxygruppe mit 1 bis 18 Kohlenstoffatomen darstellen;
R¹⁵, R¹⁶, R¹⁷, R¹⁸ und R¹⁹ jeweils unabhängig voneinander ein Wasserstoffatom, eine Hydroxylgruppe, ein Halogenatom, eine gegebenenfalls durch Hydroxyl substituierte Alkylgruppe mit 1 bis 18 Kohlenstoffatomen, eine gegebenenfalls durch Hydroxyl substituierte Estergruppe mit 1 bis 12 Kohlenstoffatomen, eine gegebenenfalls durch Hydroxyl substituierte Alkoxygruppe mit 1 bis 18 Kohlenstoffatomen oder eine gegebenenfalls durch Hydroxyl substituierte Thioalkoxygruppe mit 1 bis 18 Kohlenstoffatomen darstellen;
An⁻ ein einwertiges Anion darstellt;
die Methylenkette der gegebenenfalls durch Hydroxyl substituierten Alkylgruppe mit 1 bis 18 Kohlenstoffatomen, die durch R¹ bis R¹⁹ dargestellt wird, die gegebenenfalls durch Hydroxyl substituierte Alkoxygruppe mit 1 bis 18 Kohlenstoffatomen, die durch R¹ bis R¹⁹ dargestellt wird, und die gegebenenfalls durch Hydroxyl substituierte Thioalkoxygruppe mit 1 bis 18 Kohlenstoffatomen, die durch R¹ bis R¹⁹ dargestellt wird, gegebenenfalls durch -O- unterbrochen sein kann;
wobei mindestens eines von R¹¹ bis R¹⁴ ein Fluoratom ist; und
wobei mindestens eines von R¹ bis R¹⁹ eine Hydroxylgruppe oder eine durch Hydroxyl substituierte Thioalkoxygruppe mit 1 bis 18 Kohlenstoffatomen ist.

2. Photosäurebildner umfassend die aromatische Sulfoniumsalzverbindung nach Anspruch 1.

3. Resistzusammensetzung enthaltend den Photosäurebildner nach Anspruch 2.

4. Kationischer Polymerisationsinitiator umfassend die aromatische Sulfoniumsalzverbindung nach Anspruch 1.

5. Kationisch polymerisierbare Zusammensetzung enthaltend den kationischen Polymerisationsinitiator nach Anspruch 4.

## Revendications

1. Composé sel de sulfonium aromatique représenté par la formule générale (I) : 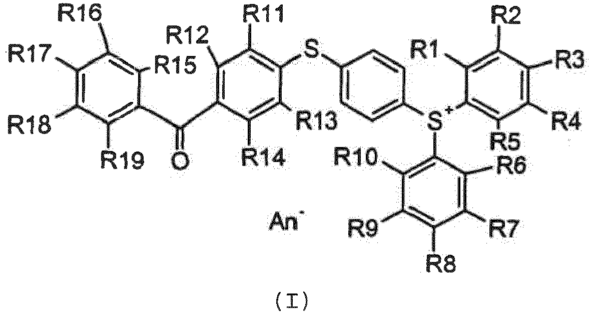
dans laquelle chacun de R¹, R², R³, R⁴, R⁵, R⁶, R⁷, R⁸, R⁹ et R¹⁰ représente indépendamment un atome d'hydrogène, un atome d'halogène, un groupe hydroxyle, un groupe alkyle éventuellement substitué par hydroxyle et ayant 1 à 18 atomes de carbone, un groupe ester éventuellement substitué par hydroxyle et ayant 1 à 12 atomes de carbone, un groupe alcoxy éventuellement substitué par hydroxyle et ayant 1 à 18 atomes de carbone, ou un groupe thioalcoxy éventuellement substitué par hydroxyle et ayant 1 à 18 atomes de carbone ;
chacun de R¹¹, R¹², R¹³ et R¹⁴ représente indépendamment un atome d'hydrogène, un atome d'halogène, un groupe hydroxyle, un groupe alkyle éventuellement substitué par hydroxyle et ayant 1 à 18 atomes de carbone, un groupe alcoxy éventuellement substitué par hydroxyle et ayant 1 à 18 atomes de carbone, ou un groupe thioalcoxy éventuellement substitué par hydroxyle et ayant 1 à 18 atomes de carbone ;
chacun de R¹⁵, R¹⁶, R¹⁷, R¹⁸ et R¹⁹ représente indépendamment un atome d'hydrogène, un groupe hydroxyle, un atome d'halogène, un groupe alkyle éventuellement substitué par hydroxyle et ayant 1 à 18 atomes de carbone, un groupe ester éventuellement substitué par hydroxyle et ayant 1 à 12 atomes de carbone, un groupe alcoxy éventuellement substitué par hydroxyle et ayant 1 à 18 atomes de carbone, ou un groupe thioalcoxy éventuellement substitué par hydroxyle et ayant 1 à 18 atomes de carbone ;
An⁻ représente un anion monovalent ;
la chaîne méthylène du groupe alkyle éventuellement substitué par hydroxyle et ayant 1 à 18 atomes de carbone représenté par R¹ à R¹⁹, du groupe alcoxy éventuellement substitué par hydroxyle et ayant 1 à 18 atomes de carbone représenté par R¹ à R¹⁹, et du groupe thioalcoxy éventuellement substitué par hydroxyle et ayant 1 à 18 atomes de carbone représenté par R¹ à R¹⁹ est éventuellement interrompue par -O- ;
au moins l'un de R¹¹ à R¹⁴ est un atome de fluor ; et
au moins l'un de R¹ à R¹⁹ est un groupe hydroxyle ou un groupe thioalcoxy substitué par hydroxyle et ayant 1 à 18 atomes de carbone.

2. Générateur de photoacide comprenant le composé sel de sulfonium aromatique selon la revendication 1.

3. Composition de réserve contenant le générateur de photoacide selon la revendication 2.

4. Amorceur de polymérisation cationique comprenant le composé sel de sulfonium aromatique selon la revendication 1.

5. Composition polymérisable par voie cationique contenant l'amorceur de polymérisation cationique selon la revendication 4.
